# EUROPÄISCHE PATENTSCHRIFT

(11) **EP 3 354 226 B1**
(45) Veröffentlichungstag und Bekanntmachung des Hinweises auf die Patenterteilung: **31.03.2021**
(21) Anmeldenummer: 18153532.9
(22) Anmeldetag: 25.01.2018
(51) Int. Cl.: A61C 7/10

(54) **DISTRAKTOR, INSBESONDERE OBERKIEFER-DISTRAKTOR**
DISTRACTOR, IN PARTICULAR UPPER JAW DISTRACTOR
DISPOSITIF SÉPARATEUR, EN PARTICULIER DISPOSITIF SÉPARATEUR POUR MÂCHOIRE SUPÉRIEURE

(30) Priorität: 27.01.2017 DE 102017101659
(43) Veröffentlichungstag der Anmeldung: 01.08.2018
(60) Teilanmeldung: 20195359.3
(73) Patentinhaber: Technische Universität Dresden, 01069 Dresden (DE)
(72) Erfinder: Botzenhart, Ute Ulrike, 01307 Dresden (DE); Gedrange, Tomasz, 01219 Dresden (DE)
(74) Vertreter: Riechelmann & Carlsohn Patentanwälte PartG mbB

(56) Entgegenhaltungen:
- EP-A2- 1 147 745
- EP-B1- 2 023 847
- WO-A1-2005/009260
- WO-A1-2016/120897
- US-A1- 2010 160 920
- US-A1- 2014 214 096

## Beschreibung

Die Erfindung betrifft einen Distraktor, insbesondere einen Oberkiefer-Distraktor. Der erfindungsgemäße Distraktor ist insbesondere zur transversalen Erweiterung eines Kiefers geeignet.

Die Maxilla stellt neben der Mandibula den größten Knochen des Viscerokraniums dar. Die Maxilla wird von zehn weiteren Schädelknochen umgeben, mit denen sie über Suturen in Verbindung steht. Der Oberkiefer selbst besteht dabei aus drei paarigen Knochen, der Prämaxilla, der Maxilla und dem Os palatinum. Diese Anteile stehen in der Medianen durch die Sutura palatina mediana in Verbindung. Die Maxilla ist aber auch an der Bildung des harten Gaumens, des Nasenbodens, am Aufbau der lateralen Nasenwand und dem Sinus maxillaris beteiligt und steht in der Medianen mit dem Vomer, der Nasenscheidewand, in Verbindung.

Die Gaumennaht (Sutura palatina mediana) und auch die übrigen Suturen des Gesichtsschädels unterliegen von der Geburt bis ins hohe Erwachsenenalter großen Veränderungen. Beim Säugling und Kleinkind besitzt die Sutura palatina mediana eine y-förmige Gestalt, ist kurz, breit und nicht verzahnt. Beim Jugendlichen hat sie bereits einen schlangenlinienartigen Verlauf, ist schmaler und verzapft sich bis ins Erwachsenenalter zunehmend (Sutura serrata = Zapfennaht). Von einer zunehmenden Verknöcherung spricht man aufgrund der sehr ausgeprägten Verhakung der Knochenanteile durch ineinandergreifende Knochenbrücken im Bindegewebe bis hin zur vollständigen Verknöcherung. Dieser Prozess vollzieht sich von dorso-nasal bis ventrooral und unterliegt erheblichen interindividuellen Schwankungen. Vor dem 40. Lebensjahr findet allerdings keine Ossifikation ventral des Canalis Incisivus statt. Mit dem 25. Lebensjahr sind ca. 25 % der Sutur verknöchert.

Die Distraktion des Oberkiefers, d. h. die Gaumennahterweiterung, ist eine der ältesten kieferorthopädischen Behandlungsmethoden und wird seit ca. 150 Jahren bei Vorliegen eines entsprechenden transversalen Defizits, eines Kreuzbisses, einem hohem schmalem Gaumen und rhinologischen Problemen, zur Erweiterung des Oberkiefers angewandt. Beschrieben wurde dieses Verfahren erstmals 1860 von Emerson Colon Angell, einem Zahnarzt aus San Francisco, der dabei eine Apparatur beschrieb, die "aus zwei sich in gegensätzlicher Richtung drehenden Schrauben mit je einem Gewinde nach rechts und links" bestand und allein an den Zahnhälsen der Prämolaren durch den Druck bei der Dehnung und der entstehenden Klemmwirkung der Apparatur bei Aktivierung befestigt war [1]. Mit dieser Apparatur erweiterte er bei einem 14 ½ Jahre alten Mädchen mit Kreuzbiss innerhalb von 2 Wochen den Oberkiefer um ca. 6 mm und nahm an, dass die Ursache des dabei auftretenden Diastema mediale eine Separation der Oberkieferhälften sei.

Heute weiß man, dass die Sutura palatina mediana sich immer infolge der Aktivierung der Dehnapparatur entgegengesetzt ihrer Verknöcherung anterior beginnend bis hin zum Os zygomaticum und zur Schädelbasis v-förmig nach posterior öffnet und daraus folgend eine Zunahme der Zahnbogenbreite resultiert, die im anterioren Bereich immer am ausgedehntesten ist. Dentoalveolär imponiert ein Diastema mediale, das sich anschließend aufgrund des Zuges, der transseptalen und supraalvelären Fasern in aller Regel spontan wieder schließt. Die nasale und orale Schleimhaut sowie der Periostüberzug bleiben in ihrer Kontinuität, Form und Funktion vollständig intakt, man spricht nach Derichsweiler von einer so genannten "Grünholzfraktur". In der Folge kommt es im Bereich der Sutur zu einer Ansammlung von Osteoblasten entlang der gestreckten kollagenen Faser, die Matrixsubstanz synthetisieren, die zu Osteoid ossifiziert und durch Einlagerung von Hydroxylapatit zu neuem Knochen reift. Dieser Prozess wird in seiner Gesamtheit als Distraktionsosteogenese bezeichnet.

Bei Kindern und Jugendlichen ist eine transversale Erweiterung des Oberkiefers aufgrund des geringen Widerstandes der umgebenden Strukturen und der geringen Verzapfung der Sutura palatina mediana sehr viel einfacher und ohne zusätzliche chirurgische Intervention möglich. CT-Untersuchungen von Habersack et al. konnten auch zeigen, dass es bei transversaler Erweiterung des Oberkiefers nicht nur zu Effekten auf die Sutura palatina mediana, sondern auch auf die umgebender Knochenanteile kommt, wie z.B. die internasale Sutur, nasomaxilläre Suturen und die frontomaxillären Suturen [2]. Aufgrund des unterschiedlichen Verknöcherungsgrades und zunehmender Festigkeit des Gesichtsskelettes ist daher mit fortschreitendem Alter eine chirurgische Schwächung vor der transversalen Distraktion des Oberkiefers angezeigt. Bei Patienten ab dem 20. Lebensjahr muss obligatorisch und ab dem 16. Lebensjahr mit großer Wahrscheinlichkeit von einer Verknöcherung der Sutur ausgegangen werden. CT-Untersuchungen über den Verknöcherungsgrad der Sutura palatina mediana von Korbmacher et al. aus dem Jahr 2007 ergaben eine signifikant höhere Knochendichte im Alter vom 25. bis 30. Lebensjahr, daher sollte jenseits des 25. Lebensjahres in keinem Falle ein konventioneller Versuch einer raschen transversalen Erweiterung mehr unternommen werden [3].

Grundsätzlich unterscheidet man unter den bekannten Distraktoren entsprechend der Indikationsstellung zahngetragene, dentale und knochengetragene, skelettale Distraktoren:
(a) Dentale Distraktoren sind zahngetragene Apparaturen, die über Verbindungselemente mit einer Dehnschraube verbunden sind. Die rasche Erweiterung des Oberkiefers erfolgt bei der Distraktion mit so hohen Kräften auf das Parodont, dass der Parodontalspalt nicht vaskularisiert und über eine Ischämie und Hyalinisierung des Knochens initial keine Zahnbewegung stattfinden kann. Die Erweiterung des Oberkiefers ist zum Zeitpunkt der Abbauphase der Hyalinisierungsprodukte und dem Beginn einer Zahnbewegung bereits abgeschlossen, d. h. die Oberkieferdehnung findet mit nur geringen dentalen Effekten statt. Mehr oder weniger ausgeprägte Nebenwirkungen treten aber bei allen dental verankerten Gaumennahterweiterungsapparaturen immer unweigerlich auf. Durch die Wirkung der Apparatur kommt es also nicht nur zu suturalen, sondern auch zu alveolären und dentalen Bewegungen. Ferner kann es durch die hohe Krafteinwirkung zu Wurzelresorptionen kommen. Nebenwirkungen dentaler Distraktoren sind: unerwünschte Zahnkippungen; eine Bissöffnung bei starken Zahnkippungen durch "hängende palatinale Höcker"; Fenestrationen der buckalen Kompakta; hohe Spannungskonzentrationen im Bereich der Schädelbasis und des Alveolarfortsatzes; iatrogene Alveolarfortsatzfrakturen; Schädelbasisfrakturen mit Nervkompression; Wurzelresorptionen, Devitalisierung von Zähnen, Extrusion von Zähnen; asymmetrische Dehnung bei interokklusalen Differenzen; Nasenseptumdeviationen; Sinusitis maxillaris; Epistaxis; ausgeprägte Rezidive. [4-9].
b) Da bei dental getragenen Distraktoren Zahnfehlstellungen erst im Anschluss an die Distraktion korrigiert werden können (was einen Umbau der Apparatur erfordert), werden vermehrt skelettal verankerte Distraktoren bevorzugt, die transversal im Gaumendach befestigt werden. M.Y. Mommaertz entwickelte mit einem nach ihm benannten Distraktor diese Idee des direkten Kraftansatzes am knöchernen Gaumen [10]. Dentale Nebeneffekte sind mit dieser Vorgehensweise nahezu auszuschließen. Von dieser Entwicklung gingen verschiedene sowohl chirurgische wie auch kieferorthopädische Therapiekonzepte aus. Die Arten der Distraktoren sind heute vielfältig, ebenso das Aktivierungsprotokoll sowie die Art und der Umfang der chirurgischen Intervention. Allerdings wird aufgrund der starken Nebeneffekte und hohen Rezidivrate dentaler und konventioneller Vorgehensweisen, der chirurgischen Intervention häufig der Vorrang gegeben. Hintergrund ist, dass bei Erwachsenen bzw. zunehmender Verknöcherung der Widerstand, den die Knochenpfeiler des Gesichtsskelettes bei Krafteinwirkung auf die beiden Knochenanteile der Maxilla dieser Dehnung entgegensetzen, so groß ist, dass die Gefahr einer iatrogenen Alveolarfortsatzfraktur oder Fraktur im Bereich der Schädelbasis besteht. Da der größte Widerstand bei Erwachsenen im lateralen Bereich der Kieferhöhle zwischen Alveolarfortsatz und Sutura zygomaticomaxillaris liegt, wird bei der chirurgisch unterstützen Gaumennahterweiterung vor Distraktion eine Schwächung vorgenommen. Diese besteht in der Regel aus einer Kombination einer modifizierten LeFort I-Osteotomie, nach Epker, Bell, Lines und Glassmann als horizontale Osteotomie in der LeFort I-Ebene im Bereich der Crista zygomaticoalveolaris beschrieben [9,11,12], und einer Hemisektion der Maxilla, wobei die Gaumennaht von vestibulär im Bereich der Spina nasalis anterior mit einem Meißel interinzisal eröffnet wird. Da eine alleinige Schwächung der Gaumennaht für das Erreichen suffizienter Resultate häufig nicht ausreichend war, entwickelten Northway und Maede sowie Zäller und Ullrich eine Technik, bei der sie im Bereich der pterygomaxillären Pfeiler der Maxilla eine laterale Osteotomie, und zur Schwächung der Gaumennaht paramedian des Septums, von palatinal aus, eine Schwächung durchführten [13]. Die Teilung der Sutur erfolgte nicht dogmatisch, sondern je nach Auffassung selektiv. Je nach weiterer Modifikation erfolgt also zusätzlich noch eine Durchtrennung am knöchernen Nasenboden lateral der Crista zygomaticoalveolaris.

Ein weiteres Verfahren, 2007 von Paulus beschrieben, umfasst eine trisegmentale Osteotomie innerhalb der Le Fort I-Ebene mit vollständiger Mobilisation der Maxilla in drei Segmente [14]. Dabei verlaufen die Osteotomielinien abweichend von oben genannten Protokollen jeweils paramedian und transalveolär im Bereich der lateralen Inzisivi und der Canini, so dass eine dreidimensionale Beeinflussung der Oberkieferdimension möglich ist. Das Verfahren bietet gegenüber der bisegmentalen Osteotomie folgende Vorteile [14]: geringe Gefahr der Wurzelverletzung bei frontalem Engstand; kleine Lücken im Bereich der seitlichen Inzisivi und der Canini mit geringerer ästhetischer Beeinträchtigung der Patienten; keine Gefahr der Septumdeviation und Verbreiterung der Collumella; dreidimensionale Beeinflussbarkeit der Oberkieferdimension.

Untersuchungen von Habersack et al. [14] zeigten bei diesem Operationsverfahren ausgeprägtere Dehnungseffekte im posterioren Bereich des Oberkiefers mit geringeren skelettale Rezidiven, was auf das Bestehenbleiben der Kontinuität der Apaturis piriformis zurückgeführt wird.

Bei den derzeit auf dem Markt befindlichen skelettalen Distraktorsystemen handelt es sich um starre Bauteile, die in unterschiedlichen Größen bzw. Längen erhältlich sind. Die Platzierung erfolgt intraoperativ, zusätzlich zu einer Schwächung der Knochenpfeiler sowie einer Schwächung im Bereich der Spina nasalis anterior, um eine möglichst ungehinderte Dehnung realisieren zu können. Die intraoperative dreidimensionale Platzierung des Distraktors kann aufgrund der unterschiedlichen Anatomie des Gaumendaches Schwierigkeiten mit sich bringen, oft nicht präzise genug in Bezug auf die Kraftvektoren erfolgen und/oder z. B. Knochenkanten oder Okklusionshindernisse können während des Distraktionsprozesses zu einer Asymmetrie oder Abweichungen in der Vertikalebene führen. Diese während des Distraktionsprozesses entstandenen Abweichungen müssen dann im Anschluss durch kieferorthopädische Zahnbewegungen ausgeglichen werden (asymmetrischer Lückenschluss, asymmetrische Intrusions- oder Extrusionsbewegungen) oder erfordern sogar im schlimmsten Falle eine weitere chirurgische Intervention (Replatzierung des Distraktors, Dysgnathieoperation).

Das derzeit üblicherweise angewandte Operationsverfahren, sieht eine Dehnung der Sutura palatina mediana vor, die altersbedingt immer von posterior nach anterior verknöchert und sich dementsprechend bei einer transversalen Erweiterung immer von anterior nach posterior (von vorn nach hinten) öffnet. Die entstehende Erweiterung wird daher immer im anterioren Kieferbereich am größten sein, was sich klinisch durch die Entstehung eines Tremas darstellt. Neben der temporären ästhetischen Einschränkung für die Patienten, ist der Distraktionsbedarf jedoch meist im posterioren Kieferbereich am größten. Die Kraftvektoren und die maximale Dehnung können zwar durch die möglichst weit dorsale Platzierung des Distraktors beeinflusst werden, der Widerstand der Hart- und Weichgewebe lässt aber bei oben beschriebener chirurgischer Vorgehensweise nur eine begrenzte posteriore Dehnung zu.

Mit Hilfe der 2007 erstmals von Paulsen beschriebenen modifizierten chirurgischen Schnittführung kann eine bessere posteriore Dehnung erreicht werden und störende Lückenbildungen im Oberkieferfrontzahnbereich können umgangen werden [14]. Die Operationstechnik sieht eine trisegmentale Schnittführung vor, bei der die Osteotomie beidseits paramedian der Sutura palatina mediana erfolgt. Es entstehen so drei Knochensegmente, wobei das zentrale Segment in der Mittellinie mit dem Vomer verbunden und fix bleibt. Diese Schnittführung hat neben den ästhetischen Vorteilen der seitlichen statt zentralen Lückenbildung auch eine wesentlich geringer Rezidivrate sowie eine maximale Dehnung im posterioren Bereich des Oberkiefers zur Folge [14]. Ein Distraktor der auf dieses Operationsverfahren abgestimmt ist, existiert derzeit nicht. Gegenwärtig erfolgt die Fixierung des Distraktors an den beiden seitlichen mobilen Kiefersegmenten.

Die Größenauswahl und Art des skelettalen Distraktors kann präoperativ an einem Gipsmodell erfolgen, sofern dies von dem behandelnden Kieferorthopäden vorliegt. In der Regel erfolgt die genaue Größenauswahl und auch Platzierung aber immer intraoperativ. Dem Chirurgen liegt zudem aus wirtschaftlichen Gründen nur eine begrenzte Anzahl an Distraktorsystemen vor. In der Regel wird ein bestimmtes Distraktorsystem präferiert. Eine intraoperativ ungünstige Platzierung kann mit den derzeit auf dem Markt befindlichen Systemen postoperativ während des Distraktionsverlaufes nicht ausgeglichen werden. Im Falle eines sehr ungünstigen Verlaufes muss der Distraktor entfernt und in einer weiteren Operation replatziert werden. Neben dem oben beschriebenen Operationsverfahren nach Paulsen, das zu einer Optimierung der Dehnungseffekte führt [14], gibt es für die oben aufgeführten Probleme keine zufriedenstellenden Lösungen.

Im Ergebnis ist der Einsatz dental getragener Distraktoren mit einer Vielzahl von Nachteilen verbunden: Es besteht das Risiko unerwünschter Zahnbewegungen, Zahnkippungen oder beides. Es kann eine Fenestrationen des Alveolarfortsatzes (bukkale Kompakta) auftreten. Hohe Spannungskonzentrationen im Bereich der Schädelbasis und Aveloarknochen sind möglich. Ferner wird der Zungenraum durch die voluminöse Apparatur eingeengt, und es besteht ein erhöhtes Kariesrisiko. Ein entstehendes Trema, d. h. eine mediane Lücke im Oberkiefer, tritt auf. Es kann zu Wurzelresorptionen kommen. Eine gleichzeitige Ausformung des Zahnbogens ist nicht möglich. Es können ausgeprägte Rezidive, eine iatrogene Alveloarfortsatzfraktur, eine Schädelbasisfraktur sowie Nervverletzugen auftreten. Außerdem besteht die Gefahr einer asymmetrischen Dehnung bei unkontrollierter Öffnung der Suturen (z. B. Septumdeviation). Der Zugang für den Chirurgen ist erschwert, da die Eingliederung der Dehnungsapparatur präoperativ erfolgt. Schließlich besteht die Gefahr einer Lockerung des Distraktors, und zwar intraoperativ, während der Konsolidierungsphase oder beides, wobei die Replatzierung des Distraktors schwierig ist.

Bislang bekannte skelettal verankerte Distraktoren weisen eine Passungenauigkeit auf, so dass sie auf die anatomischen Merkmale des Patienten nicht zufriedenstellend abgestimmt sind. Daraus folgt eine Varianz bei der Positionierung, die vor allem bei schmalem hohen Gaumendach auftritt. Nachjustierungen sind nicht möglich. Die Kraftvektoren sind nicht vollständig vorhersehbar. Insbesondere bei einer asymmetrischen Platzierung des Distraktors besteht die Gefahr unerwünschter Abweichungen, z. B. eine "hängende Okklusionsebene" oder eine asymmetrische Dehnung. Es können Irritationen von Weichgeweben im transgingivalen Bereich auftreten, was die vorzeitige Entfernung des Distraktors während der Konsolidierungszeit bei rezidivieren Entzündungen notwendig machen kann. Auf ein entstehendes Trema, d. h. eine mediane Lücke im Oberkiefer, bei Einsatz bekannter Distraktoren und/oder Operationstechniken wurde bereits hingewiesen.

Aus US 2010/0160920 A1 und WO 2005/009260 A1 sind Oberkieferdistraktoren zur transversalen Erweiterung des Oberkiefers bekannt. Diese Distraktoren ermöglichen jedoch keine Fixierung des anterioren Segmentes, das nach der von Paulsen et al. beschriebenen trisegmentalen Operation des Oberkiefers stationär bleiben soll. Aus EP 1 147 745 A2 ist eine Vorrichtung für die Gaumenerweiterung bekannt.

Aufgabe der Erfindung ist es, die Nachteile nach dem Stand der Technik zu beseitigen. Es soll insbesondere ein Distraktor angegeben werden, der eine verbesserte Passgenauigkeit besitzt.

Diese Aufgabe wird durch die Merkmale von Anspruch 1 gelöst. Zweckmäßige Ausgestaltungen der Erfindungen ergeben sich aus den Merkmalen der Unteransprüche.

Nach Maßgabe der Erfindung ist ein Distraktor vorgesehen, der insbesondere zur transversalen Erweiterung eines Kiefers eines Patienten bestimmt ist. Der Distraktor weist eine Hülse, in der entlang einer Längsachse bewegbare Expansionselemente angeordnet sind, und erste Fixierungselemente zum Befestigen des Distraktors am Kiefer eines Patienten auf. Die ersten Fixierungselemente sind über erste Kopplungselemente mit den Expansionselementen verschwenkbar verbunden. Zwischen den ersten Fixierungselementen und den ersten Kopplungselementen und zwischen den ersten Kopplungselementen und den Expansionselementen sind Gelenke ausgebildet. Die ersten Kopplungselemente sind mit den Expansionselementen jeweils über ein Kugelgelenk, das als zweites Kugelgelenk bezeichnet ist, verbunden.

Der erfindungsgemäße Distraktor weist somit zumindest zwei Fixierungselemente auf, nämlich die als "erste Fixierungselemente" bezeichneten Fixierungselemente. Es kann vorgesehen sein, dass der erfindungsgemäße Distraktor ein weiteres Fixierungselement zum Befestigen des Distraktors am Kiefer eines Patienten aufweist. Dieses weitere Fixierungselement wird als zweites Fixierungselement bezeichnet. Die ersten Fixierungselemente und das zweite Fixierungselement werden im Folgenden gemeinsam als Fixierungselemente bezeichnet. Die Fixierungselemente können gleich sein. Insbesondere können die Fixierungselemente die gleiche Form aufweisen und aus dem gleichen Material bestehen. Bei den Fixierungselementen kann es sich um Schrauben handeln. Der erfindungsgemäße Distraktor kann Distraktorplatten aufweisen, durch die die Fixierungselemente zur knöchernen Fixierung der Distraktorplatten am Oberkiefer, genauer am knöchernen Gaumen des Oberkiefers, geführt werden. Die Distraktorplatten sind zweckmäßigerweise gewölbt und an das Gaumendach angepasst.

Vorzugsweise ist das zweite Fixierungselement von einem zweiten Kopplungselement an dem Distraktor gehalten. Das zweite Fixierungselement kann an der Hülse verschwenkbar befestigt sein. Das zweite Fixierungselement kann so angeordnet sein, dass es um die Hülse verdrehbar ist. Der erfindungsgemäße Distraktor kann somit zusätzlich zu den beiden ersten Kopplungselementen ein zweites Kopplungselement aufweisen. Die ersten Kopplungselemente und das zweite Kopplungselement werden im Folgenden gemeinsam als Kopplungselemente bezeichnet. Die Kopplungselemente können gleich sein. Die Fixierungselemente können einander gleichen. Insbesondere können die Fixierungselemente die gleiche Form aufweisen und aus dem gleichen Material bestehen. Während die beiden ersten Kopplungselemente sich im Wesentlichen in einer Richtung erstrecken, die auf der Längsachse der Hülse liegt, sollte sich das zweite Kopplungselement in eine Richtung erstrecken, die im Wesentlichen orthogonal zur Längsachse der Hülse liegt.

Es kann vorgesehen sein, dass die ersten Fixierungselemente mit den ersten Kopplungselementen jeweils über ein Kugelgelenk, das als erstes Kugelgelenk bezeichnet ist, verbunden sind. Dazu kann das erste Fixierungselement einen Gelenkkopf aufweisen, der in einer an dem ersten Kopplungselement ausgebildeten Kugelpfanne gelagert ist. Die ersten Kopplungselemente sind mit den Expansionselementen jeweils über das zweite Kugelgelenk verbunden. Dazu kann das jeweilige erste Kopplungselement einen Gelenkkopf aufweisen, der in einer an dem Expansionselement ausgebildeten Kugelpfanne gelagert ist.

In der gleichen Weise kann das zweite Fixierungselement mit dem zweiten Kopplungselement über ein drittes Kugelgelenk verbunden sein. Dazu kann das zweite Fixierungselement einen Gelenkkopf aufweisen, der in einer an dem zweiten Kopplungselement ausgebildeten Kugelpfanne gelagert ist. In einer Ausführungsform der Erfindung ist das zweite Kopplungselement nicht mit einem Expansionselement verbunden. Es kann vorgesehen sein, dass die Hülse einen Träger hält, wobei das zweite Kopplungselement das zweite Fixierungselement mit dem Träger verbindet. Der Träger kann somit die Befestigung des zweiten Kopplungselementes an der Hülse ermöglichen. Der Träger kann um die Längsachse der Hülse drehbar sein. Das zweite Kopplungselement kann mit dem Träger über ein viertes Kugelgelenk verbunden sein. Dazu kann das zweite Kopplungselement einen Gelenkkopf aufweisen, der in einer an dem Träger ausgebildeten Kugelpfanne gelagert ist. Der Träger ist vorzugsweise auf der Hülse verschiebbar. Die Hülse kann, wie nachstehend beschrieben wird, beispielsweise als Mehrkant ausgebildet sein, um eine Verschiebung des Trägers entlang der Längsachse der Hülse oder eine Drehung des Trägers um die Längsachse der Hülse bewirken zu können.

Die ersten, zweiten, dritten und vierten Kugelgelenke können einander gleichen. Sie werden im Folgenden gemeinsam als Kugelgelenke bezeichnet. Zur Fixierung der Gelenkköpfe in den Kugelpfannen der Kugelgelenke können Feststellelemente, beispielsweise Schrauben wie Stell- oder Kantschrauben vorgesehen sein. Dazu können in den Seitenwänden der Kugelpfannen Bohrungen, beispielsweise Versenkbohrungen, ausgebildet sein, die ein Innengewinde aufweisen, durch das die Schraube mit einem korrespondieren Außengewinde geführt werden kann. Die Schraube kann dann in die Bohrung eingeschraubt werden, wodurch sie mit einer Stirnseite gegen den Gelenkkopf gepresst wird. Auf diese Weise kann der Gelenkkopf in der Kugelpfanne fixiert werden. Zum Lösen der Fixierung kann die Schraube wieder aus der Bohrung geschraubt werden.

Die Hülse kann beispielsweise ein prismatischer oder zylindrischer Körper sein, der eine Durchgangsöffnung aufweist, die sich von einer Stirnseite des prismatischen oder zylindrischen Körpers zu der gegenüberliegenden Stirnseite dieses Körpers erstreckt. Die Durchgangsöffnung weist vorzugsweise einen kreisförmigen Querschnitt auf. Die Längsachse der Durchgangsöffnung liegt zweckmäßigerweise auf der Längsachse der Hülse. Die Hülse weist somit eine Außenseite und eine Innenseite auf. Die Innenseite begrenzt die Durchgangsöffnung. An der Außenseite kann sich der Träger für das zweite Kopplungselement und das zweite Fixierungselement befinden.

Die Expansionselemente weisen vorzugsweise einen zylinderförmigen Abschnitt mit einem kreisförmigen Querschnitt auf. Die Mantelfläche des zylinderförmigen Abschnittes ist zweckmäßigerweise komplementär zu der Innenseite der Hülse. Zur Führung der Expansionselemente können Führungselemente vorgesehen sein. Dabei kann für jedes Expansionselement ein gesondertes Führungselement vorgesehen sein. Die Führungselemente sollen eine Verschiebung der Expansionselemente entlang der Längsachse der Hülse ermöglichen und eine Verdrehung der Expansionselemente um die Längsachse der Hülse verhindern. Bei dem Führungselement kann es sich um eine Nut handeln, in die ein Steg eingreifen kann.

Beispielsweise können in der Mantelfläche des zylinderförmigen Abschnittes eine oder mehreren Nuten ausgebildet sein, in die entsprechende Stege eingreifen, die an der Innenseite der Hülse in der Durchgangsöffnung ausgebildet sind. Der oder die Stege sind vorzugsweise parallel zur Längsachse der Hülse ausgebildet. Ebenso verlaufen die Nut oder die Nuten vorzugsweise parallel zur Längsachse der Hülse. Es kann alternativ vorgesehen sein, dass der oder die Stege an der Mantelfläche des zylinderförmigen Abschnittes und die Nut oder die Nuten in der Innenseite der Hülse ausgebildet sind. Nut und Steg ermöglichen eine Verschiebung der Expansionselemente entlang der Längsachse der Hülse, verhindern aber eine Drehung der Expansionselemente um die Längsachse der Hülse. Die Nut kann somit als Führungsnut für das Expansionselement dienen.

Die Expansionselemente können zusätzlich zu dem zylinderförmigen Abschnitt einen zweiten Abschnitt aufweisen, der an den zylinderförmigen Abschnitt angrenzt. Der zweite Abschnitt weist keinen kreisförmigen Querschnitt auf, dessen Mittelpunkt auf der Längsachse der Hülse liegt. Vorzugsweise entspricht der Querschnitt des zweiten Abschnittes einem Kreissegment, wobei der Kreisbogen des Kreissegmentes den gleichen Radius wie der zylinderförmige Abschnitt besitzt. Die Mantelfläche des zweiten Abschnittes weist somit denselben Krümmungsradius wie der zylinderförmige Abschnitt auf und verläuft vorzugsweise fluchtend zu der Mantelfläche des zylinderförmigen Abschnittes. Die Segmenthöhe des Kreissegmentes ist vorzugsweise geringer als sein Radius. Der Radius erstreckt sich dabei von der Längsachse der Hülse bis zum Kreisbogen des Kreissegmentes. In der Mantelfläche des zweiten Abschnittes kann eine Nut ausgebildet sein, die fluchtend zu einer Nut im zylinderförmigen Abschnitt verläuft und an diese angrenzt. Der Steg, der an der Innenseite der Hülse in der Durchgangsöffnung ausgebildet ist, greift in diesem Fall in die Nut des zylinderförmigen Abschnittes und des zweiten Abschnittes ein. Alternativ kann an der Mantelfläche des zylinderförmigen Abschnittes ein Steg ausgebildet sein, der fluchtend zu einem Steg im zylinderförmigen Abschnitt verläuft und an diesen angrenzt. In diesem Fall ist die Nut in der Innenseite der Hülse ausgebildet. Der zylinderförmige Abschnitt und der zweite Abschnitt sind vorzugsweise einstückig ausgebildet.

Vorzugsweise gleichen die beiden Expansionselemente einander. Dabei sind sie vorzugsweise so angeordnet, dass sich die zweiten Abschnitte der Expansionselemente in der Hülse gegenüberliegen. An den Stirnseiten der zylinderförmigen Abschnitte der beiden Expansionselemente, die den zweiten Abschnitten abgewandt sind, können die ersten Kopplungselemente angeordnet sein. Dazu können in diesen Stirnseiten der zylinderförmigen Abschnitte die Kugelpfannen ausgebildet sein, in die die Gelenkköpfe der ersten Kopplungselemente eingreifen. Aufgrund des kreissegment-förmigen Querschnittes der zweiten Abschnitte der Expansionselemente weisen die zweiten Abschnitte jeweils eine gekrümmte Fläche, ihre Mantelfläche, und eine flache Fläche, die im Folgenden als Flachseite bezeichnet wird, auf. Die Stirnseite des zylinderförmigen Abschnittes, an die der zweite Abschnitt des Expansionselementes angrenzt, liegt teilweise frei, weil die Segmenthöhe des zweiten Abschnittes geringer als der Durchmesser und auch geringer als der Radius des zylinderförmigen Abschnittes ist. Zwischen dem zylinderförmigen Abschnitt und dem zweiten Abschnitt eines Expansionselementes entsteht somit ein Überstand.

Vorzugsweise liegen sich die beiden Expansionselemente mit den Flachseiten ihrer zweiten Abschnitte gegenüber. Dabei kann die Stirnseite des zweiten Abschnittes des ersten Expansionselementes, die dem zylinderförmigen Abschnitt dieses Expansionselementes abgewandt ist, dem Überstand des zweiten Expansionselementes zugewandt sein. Die Stirnseite des zweiten Abschnittes des zweiten Expansionselementes, die dem zylinderförmigen Abschnitt dieses Expansionselementes abgewandt ist, ist dann dem Überstand des ersten Expansionselementes zugewandt. Zwischen den Flachseiten der beiden Expansionselemente befindet sich dabei ein Zwischenraum.

Es kann vorgesehen sein, dass in der Hülse zumindest ein Federelement zwischen einander zugewandten Stirnseiten der Expansionselemente angeordnet ist. Weisen die Expansionselemente jeweils einen zylinderförmigen Abschnitt und einen zweiten Abschnitt auf, so kann vorgesehen sein, dass jeweils ein Federelement zwischen dem Überstand des einen Expansionselementes und der dem Überstand zugewandten Stirnseite des anderen Expansionselementes angeordnet ist. Das oder die Federelemente sollen das Verschieben der Expansionselemente, insbesondere bei dessen Aktivierung mittels des nachstehend beschriebenen Stellelementes, in der Hülse unterstützen. Bei dem oder den Federelementen kann es sich um Druckfedern handeln.

Der Distraktor kann ein Stellelement zur Aktivierung und Verschiebung der Expansionselemente entlang der Längsachse der Hülse aufweisen. Das Stellelement kann in den Zwischenraum eingreifen, der zwischen den beiden Flachseiten der zweiten Abschnitte der Expansionselemente ausgebildet ist. Das Stellelement kann beispielsweise eine Schraube, ein Zahnrad oder ein Doppelzahnrad sein. Mittels des Stellelementes können die Expansionselemente in gegengesetzter Richtung entlang der Längsachse der Hülse verschoben werden. Die Drehachse des Stellelementes verläuft vorzugsweise orthogonal zur Längsachse der Hülse. Ist das Stellelement eine Schraube, so kann die Schraube ein Gewinde aufweisen, das in eine Zahnreihe, die an der Flachseite des ersten Expansionselementes ausgebildet ist, und eine Zahnreihe, die an der Flachseite des zweiten Expansionselementes ausgebildet ist, eingreift. Ist das Stellelement ein Zahnrad, so kann das Zahnrad ein Gewinde aufweisen, das in eine Zahnreihe, die an der Flachseite des ersten Expansionselementes ausgebildet ist, und eine Zahnreihe, die an der Flachseite des zweiten Expansionselementes ausgebildet ist, eingreift. Handelt es sich bei dem Stellelement um ein Doppelzahnrad, so kann das Doppelzahnrad einen ersten Zahnkranz, der in eine Zahnreihe eingreift, die an der Flachseite des ersten Expansionselementes ausgebildet ist, und einen zweiten Zahnkranz aufweisen, der in eine Zahnreihe eingreift, die an der Flachseite des anderen Expansionselementes ausgebildet ist. Die Zahnreihen, die an den Flachseiten der Expansionselemente ausgebildet sind, erstrecken sich vorzugsweise parallel zur Längsachse der Hülse. Das Stellelement kann einen Stift aufweisen, der sich durch eine Zugangsöffnung, die im Mantel der Hülse ausgebildet ist, erstreckt und dem Anwender des erfindungsgemäßen Distraktors eine Drehung des Stellelementes um dessen Drehachse ermöglicht. Das Stellelement kann in der Hülse gelagert sein.

Der Träger, der von der Hülse gehalten wird und über das zweite Kopplungselement mit dem zweiten Fixierungselement verbunden ist, kann auf die Hülse aufgesteckt sein. Der Träger sollte den Zugang zu dem Stellelement nicht behindern. Der Träger ist zweckmäßigerweise in einer bestimmten Position auf der Hülse lösbar arretierbar. Dazu kann der Träger über eine Klemmverbindung an der Hülse lösbar arretiert sein. Beispielsweise kann es sich beim dem Träger um eine Klemme oder einen Halbring handeln, dessen Innenseite an der Außenseite der Hülse anliegt. An der Außenseite des Halbringes kann die Kugelpfanne ausgebildet sein, die den Kugelkopf des zweiten Kopplungselementes aufnehmen kann. Es kann vorgesehen sein, dass an der Außenseite der Hülse Rastmittel ausgebildet sind, die eine Justierung des Trägers an der Hülse ermöglichen. Mittels der Rastmittel kann der Träger in einer vom Anwender gewünschten Position an der Hülse gehalten werden. Im eingerasteten Zustand ist der Träger dabei an der Hülse fixiert. Wird die Rastverbindung gelöst, kann der Träger um die Längsachse der Hülse gedreht werden. Ist die Hülse ein prismatischer Körper, so kann deren Außenfläche mehrere Kanten aufweisen, beispielsweise vier, fünf, sechs, sieben, acht oder mehr Kanten. Die Hülse kann dann als Mehrkant angesehen werden. Die Kanten können als Rastmittel zur Festlegung der Position des Trägers zur Hülse dienen.

In der vorliegenden Erfindung wird unter einem Halbring ein Körper verstanden, der einen im Wesentlichen C-förmigen Querschnitt hat. An der Außenseite der Basis kann die Kugelpfanne ausgebildet sein. Die beiden Schenkel ermöglichen eine sicherere und lösbare Arretierung des Halbrings an der Hülse.

Die Komponenten des Distraktors bestehen vorzugsweise aus dem gleichen Material, beispielsweise Titan oder einer Titanlegierung.

Der erfindungsgemäße Distraktor erlaubt eine bessere intraoperative Platzierung, insbesondere aufgrund der Mobilität der Kugelgelenke. Dadurch ist eine bessere Anpassung an die Anatomie des Oberkiefers möglich. Der erfindungsgemäße Distraktor ermöglicht ferner eine postoperative Nachjustierung in allen drei Raumebenen, wenn dies während des Distraktionsprozesses notwendig wird. Eine Replatzierung, die einen weiteren chirurgischen Eingriff erforderlich machen würde, kann so vermieden werden.

Weist der Distraktor neben den beiden ersten Fixierungselementen ein zweites Fixierungselement auf, so kann der Distraktor mittels des zweiten Fixierungselementes im anterioren Segment des Oberkiefers verankert werden. Wird der Distraktor in einer Führungsebene stabilisiert, so dass Vertikalabweichungen der seitlichen Segmente während der Distraktion verhindert werden können. Weist der erfindungsgemäße Distraktor hingegen nur die beiden ersten Fixierungselemente auf, so sind diese vorzugsweise in den lateralen Segmenten des Oberkiefers verankert.

Für eine Anpassung in allen drei Raumebenen sowie eine Nachjustierung während der Distraktion ist es vorteilhaft, wenn die Gelenke zwischen den beiden ersten Fixierungselementen und den ersten Kopplungselementen sowie den ersten Kopplungselementen und den Expansionselementen Kugelgelenke sind. Weist der erfindungsgemäße Distraktor ein zweites Fixierungselement auf, so ist es für diese Zwecke ebenso vorteilhaft, wenn die Gelenke zwischen dem zweiten Fixierungselement und dem zweiten Kopplungselement sowie dem zweiten Kopplungselement und dem Träger Kugelgelenke sind. Ist ein Fixierungselement über ein Kugelgelenk mit einem Kopplungselement und das Kopplungselement mit einem Expansionselement oder dem Träger ebenfalls über ein Kugelelement verbunden, so wird diese Art der Verbindung im Folgenden auch als Kugeldoppelgelenk bezeichnet.

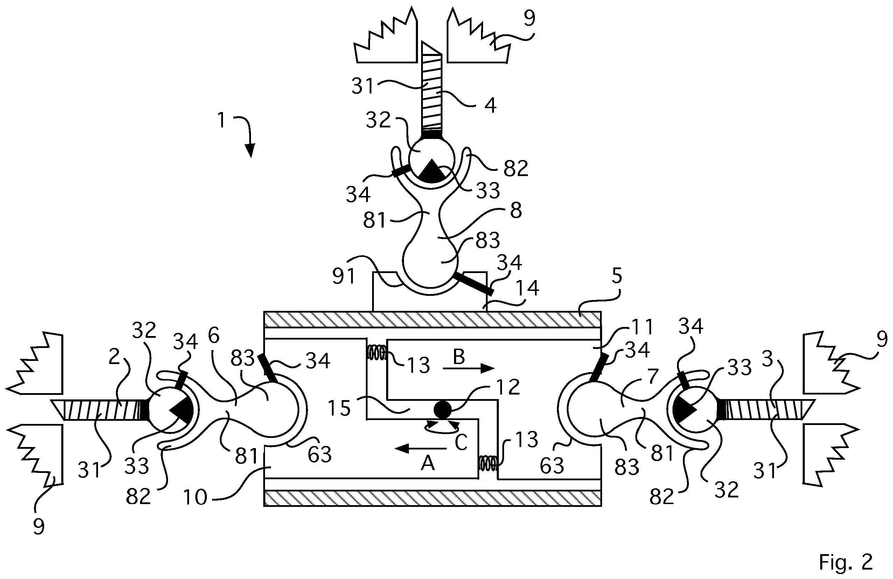

Der erfindungsgemäße Distraktor kann mit drei Kugeldoppelgelenken ausgestattet sein, wenn er zwei erste Fixierungselemente und ein zweites Fixierungselement aufweist. In diesem Fall kann der erfindungsgemäße Distraktor besonders vorteilhaft in Kombination mit der Operationstechnik nach Paulsen (siehe insbesondere [14], Fig. 2 und die zugehörige Beschreibung) eingesetzt werden. Dabei werden die beiden ersten Fixierungselemente in den lateralen Segmenten des Oberkiefers verankert, während das zweite Fixierungselement in dem zentralen Segment des Oberkiefers verankert wird. Gemäß dieser Operationstechnik sollen mittels des Distraktors die beiden lateralen Segmente in entgegengesetzte Richtungen voneinander wegbewegt werden, um eine transversale Erweiterung des Oberkiefers zu erreichen, während die Lage des zentralen Segmentes nicht verändert werden soll und somit fix ist. Das zentrale Segment liegt anterior am Oberkiefer und kann somit auch als anteriores Segment bezeichnet werden. Der erfindungsgemäße Distraktor erlaubt damit eine sichere Steuerung der Segmente mit einer geringen Rezidivwahrscheinlichkeit. Der erfindungsgemäße Distraktor ist aber auch dann vorteilhaft anwendbar, wenn er kein zweites Fixierungselement, sondern nur die beiden ersten Fixierungselemente aufweist.

Der erfindungsgemäße Distraktor kann nur die beiden ersten Fixierungselemente, die über die ersten Kopplungselemente mit den beiden Expansionselementen verbunden sind, aufweisen. Diese Ausführungsform wird im Folgenden auch als Zweifach-Doppelgelenk-Distraktor bezeichnet. Weist der erfindungsgemäße Distraktor zusätzlich zu den beiden ersten Fixierungselementen auch das zweite Fixierungselement auf, das zur Befestigung des Distraktors im zentralen Segment des Oberkiefers dienen kann, so wird die Ausführungsform im Folgenden auch als Dreifach-Doppelgelenk-Distraktor bezeichnet. Das zweite Fixierungselement wird mittels des zweiten Kopplungselementes an der Hülse gehalten. Das zweite Fixierungselement und das zweite Kopplungselement können in Bezug auf ihre Lage am Oberkiefer als anteriores Doppelgelenk bezeichnet werden. Der erfindungsgemäße Zweifach-Doppelgelenk-Distraktor weist kein anteriores Doppelgelenk auf.

Der erfindungsgemäße Distraktor, insbesondere der Zweifach-Doppelgelenk-Distraktor, kann in Kombination mit anderen Operationsverfahren als dem Operationsverfahren nach Paulsen angewendet werden. Auch der Zweifach-Doppelgelenk-Distraktor ermöglicht eine Nachjustierung zur Steuerung der lateralen Segmente während der Distraktion. Die Anwendung des erfindungsgemäßen Distraktors ist in Kombination mit dem Operationsverfahrens nach Paulsen besonders vorteilhaft, weil der Distraktor an diese Operationstechnik angepasst ist, indem er eine dreidimensionale Nachjustierung nach einer trisegmentalen Operation ermöglicht. Der erfindungsgemäße Distraktor erlaubt neben einer Höhenverstellbarkeit auch einen Symmetrieausgleich, insbesondere zwischen den Doppelgelenken, die mit den Expansionselementen verbunden sind.

Die intraoperative Platzierung ist exakter und eindeutiger möglich. Die Kraftvektoren können postoperativ durch Nachjustierung des Distraktors optimiert werden. Der Distraktor kann z. B. in der Höhe verändert werden, wenn die Dehnung der lateralen Segmente des Oberkiefers soweit fortgeschritten ist, dass es transversal zu Druckstellen kommen könnte. Nebenwirkungen, die durch Zahnbewegungen oder gegebenenfalls weitere chirurgische Eingriffe korrigiert werden müssten, können wesentlich reduziert werden.

Die Hülse des erfindungsgemäßen Distraktors kann gemeinsam mit den beiden Expansionselementen als Distraktionszylinder bezeichnet werden. Die erfindungsgemäß vorgesehene Kombination dreier Doppelgelenke mit einem solchen Distraktionszylinder ist besonders vorteilhaft.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Darstellung einer Ausführungsform eines erfindungsgemäßen Distraktors an einem Oberkiefer;
- Fig. 2: eine schematische Darstellung der Komponenten der in Fig. 1 gezeigten Ausführungsform;
- Fig. 3: eine perspektivische Darstellung der Hülse der in Fig. 1 gezeigten Ausführungsform eines erfindungsgemäßen Distraktors;
- Fig. 4: schematische Darstellungen eines Expansionselementes der in Fig. 1 gezeigten Ausführungsform eines erfindungsgemäßen Distraktors (Fig. 4a: perspektivische Darstellung; Fig. 4b: Ansicht von vorn; Fig. 4c: Seitenansicht; Fig. 4d: Ansicht von hinten);
- Fig. 5: schematische Darstellungen der Hülse und der darin angeordneten Expansionselemente der in Fig. 1 gezeigten Ausführungsform eines erfindungsgemäßen Distraktors (Fig. 5a: Ansicht von vorn; Fig. 5b: Schnitt entlang Schnittlinie A--A in Fig. 5a; Fig. 5c: Ansicht von hinten; Fig. 5d: Schnitt entlang Schnittlinie B--B in Fig. 5b);
- Fig. 6: eine schematische Darstellung eines Fixierungselementes und eines Gelenkkopfes der in Fig. 1 gezeigten Ausführungsform eines erfindungsgemäßen Distraktors;
- Fig. 7: schematische Darstellungen des Trägers der in Fig. 1 gezeigten Ausführungsform eines erfindungsgemäßen Distraktors (Fig. 7a: Ansicht von vorn, Fig. 7b: Ansicht von oben);
- Fig. 8: eine schematische Darstellung eines Trägers, der auf eine in Fig. 3 gezeigte Hülse aufgesteckt ist; und
- Fig. 9: einen schematischen Querschnitt des Gaumens zur Veranschaulichung der Höhenverstellbarkeit der in Fig. 1 gezeigten Ausführungsform eines erfindungsgemäßen Distraktors.

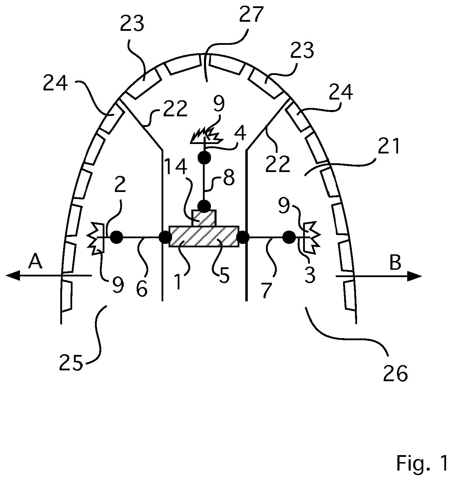

Die in den Figuren 1 bis 9 gezeigte Ausführungsform des erfindungsgemäßen Distraktors 1 weist drei Fixierungselemente, nämlich die beiden ersten Fixierungselemente 2, 3 und das zweite Fixierungselement 4 auf. Mittels der Fixierungselemente, 2, 3 und 4, bei denen es sich um selbstschneidende Schrauben, beispielsweise Knochenschrauben handeln kann, ist der Distraktor 1 am knöcherne Gaumen des Oberkiefers 21 befestigt (siehe Fig. 1). Dazu weist der Distraktor 1 Distraktorplatten 9 auf, die am Oberkiefer 21 anliegen und jeweils eine Öffnung aufweisen, durch die die Fixierungselemente, 2, 3 und 4 geführt sind. In Fig. 1 sind Osteotomielinien 22 nach Paulsen eingezeichnet. Die Osteotomielinien 22 sind horizontale, paramedian palatale und transalveolare Osteotomielinien zwischen den lateralen Schneidezähnen und den Eckzähnen, wie sie unter anderem in [14], Fig. 2a, S. 2281, gezeigt sind. Mittels der Osteotomielinien 22 wird der knöcherne Gaumen 21 in drei Knochensegmente 25, 26, 27 getrennt. Zur transversalen Erweiterung des Oberkiefers sollen die lateralen Segmente 25, 26 in transversaler Richtung (Pfeile A und B) voneinander weg bewegt werden, während das zentrale Segment 27, das zwischen den beiden lateralen Segmenten 25, 26 liegt, fix ist. Dazu sind die ersten Fixierungselemente 2, 3 in die lateralen Segmente 25, 26 eingeschraubt, während das zweite Fixierungselement 4 in das zentrale Segment 27 einschraubt ist. Der in Fig. 1 gezeigte Distraktor 1 ist ein Dreifach-Kugelgelenk-Distraktor. Er weist somit drei Kugeldoppelgelenke auf. Das anteriore Gelenk, zu dem das zweite Fixierungselement 4 und das zweite Kopplungselement 8 gehören, ermöglicht eine Fixierung des Distraktors 1 im anterioren Gaumendach des Oberkiefers 21. Gemäß der Osteotomie nach Paulsen ist das zentrale Segment während der Distraktion fix und stabilisiert den Distraktor 1 so in der Distraktionsebene. Das zentrale Segment liegt, wie in Fig. 1 zu erkennen ist, anterior.

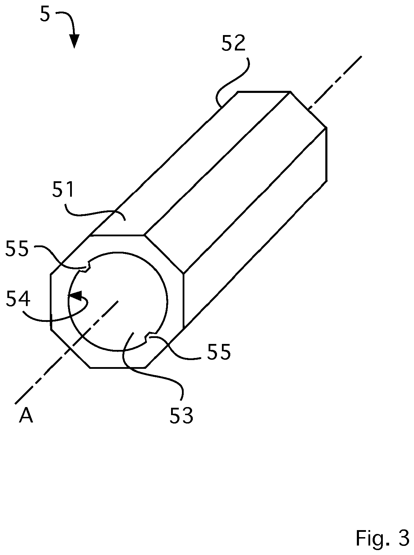

In Fig. 2 sind die Komponenten der Ausführungsform des Distraktors 1 in schematischer Weise dargestellt. Die Hülse 5, die die Expansionselemente 10, 11 aufnimmt, weist einen Querschnitt auf, der einem regelmäßigen Achteck entspricht (siehe Fig. 3). Der Mittelpunkt des regelmäßigen Achteckes liegt auf der Längsachse A der Hülse. An der Außenseite 51 sind Kanten 52 ausgebildet, die parallel zur Längsachse A der Hülse 5 verlaufen. Die Hülse 5 weist eine Durchgangsöffnung 53 auf, die sich von der einen Stirnseite der Hülse zu deren gegenüberliegenden, parallel verlaufenden Stirnseite erstreckt. Die Durchgangsöffnung 53 besitzt einen kreisförmigen Querschnitt und wird von der Innenseite 54 der Hülse begrenzt. An der Innenseite 54 können ein oder mehrere Führungsmittel, beispielsweise eine oder mehrere Stege 55, ausgebildet sein, die sich parallel zur Längsachse A der Hülse 5 erstrecken und die Verschiebung der Expansionselemente 10, 11 entlang der Längsachse A der Hülse 5, ermöglichen. In der gezeigten Ausführungsform des Distraktors 1 sind zwei Stege 55 vorgesehen. Diese beiden Stege können um 180° versetzt, bezogen auf den Querschnitt der Durchgangsöffnung 53, angeordnet sein. Die beiden Stege 55 erstrecken sich über die gesamte Länge der Hülse 5.

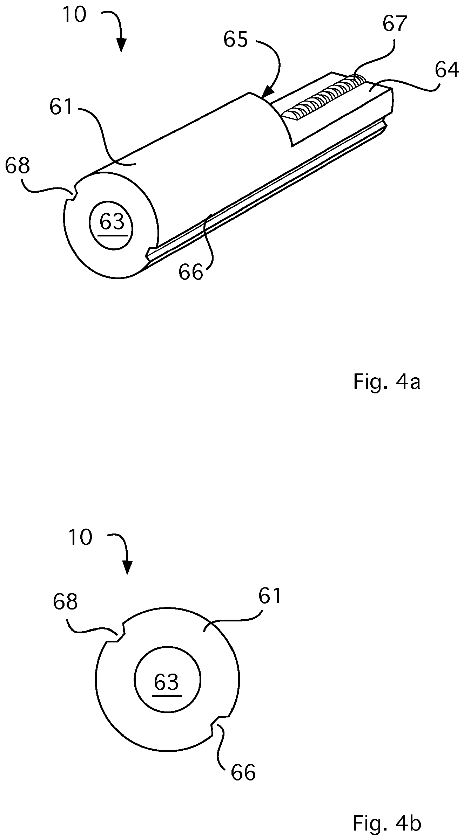

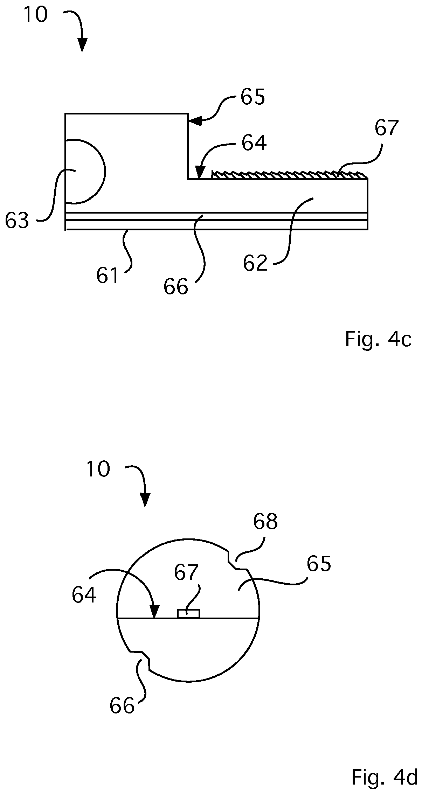

In Fig. 4 ist ein Expansionselement 10 gezeigt. Das Expansionselement 10 besitzt einen zylinderförmigen Abschnitt 61 und einen zweiten Abschnitt 62, der an eine Stirnseite des zylinderförmigen Abschnittes 61 angrenzt. In der anderen Stirnseite des zylinderförmigen Abschnittes 61 ist eine Kugelpfanne 63 für das erste Kopplungselement 6 ausgebildet. Der zweite Abschnitt 62 weist einen Querschnitt in Form eines Kreissegmentes auf. Dabei hat der Kreisbogen des Kreissegmentes den gleichen Radius wie der zylinderförmige Abschnitt 61. Die Mantelfläche des zweiten Abschnittes verläuft fluchtend zu der Mantelfläche des zylinderförmigen Abschnittes 61. Die Segmenthöhe des Kreissegmentes ist geringer als sein Radius. Der Radius erstreckt sich dabei von der Längsachse A der Hülse 5 bis zum Kreisbogen des Kreissegmentes. Aufgrund seines kreissegment-förmigen Querschnittes weist der zweite Abschnitt 62 eine gekrümmte Fläche, seine Mantelfläche, und eine flache Fläche, die sogenannte Flachseite 64, auf. Die Stirnseite des zylinderförmigen Abschnittes 61, an die der zweite Abschnitt 62 des Expansionselementes 10 angrenzt, liegt unter Ausbildung eines Überstandes 65 teilweise frei. Das Expansionselement 10 weist eine erste Nut 66 und eine zweite Nut 67 auf, in die die Stege 55 der Hülse 5 eingreift. Die erste Nut 66 erstreckt sich parallel zur Längsachse A der Hülse über die Mantelfläche des zylinderförmigen Abschnittes 61 und die Mantelfläche des zweiten Abschnittes 62. Die zweite Nut 67 erstreckt sich parallel zur Längsachse A der Hülse über die Mantelfläche des zylinderförmigen Abschnittes 61. Die beiden Nuten 66, 67 können um 180° versetzt, bezogen auf den Querschnitt des zylinderförmigen Abschnittes 61, angeordnet sein. An der Flachseite 64 ist eine Zahnreihe 67 ausgebildet, wobei die Zahnreihe parallel zur Längsachse A der Hülse 5 verläuft. Das Expansionselement 11 hat den gleichen Aufbau wie das Expansionselement 10. Es kann in der Hülse 5 spiegelverkehrt zum Expansionselement 10 angeordnet sein. Die Merkmale des Expansionselementes 11 sind mit gleichen Bezugszeichen bezeichnet, wie die entsprechenden Merkmale des Expansionselementes 10.

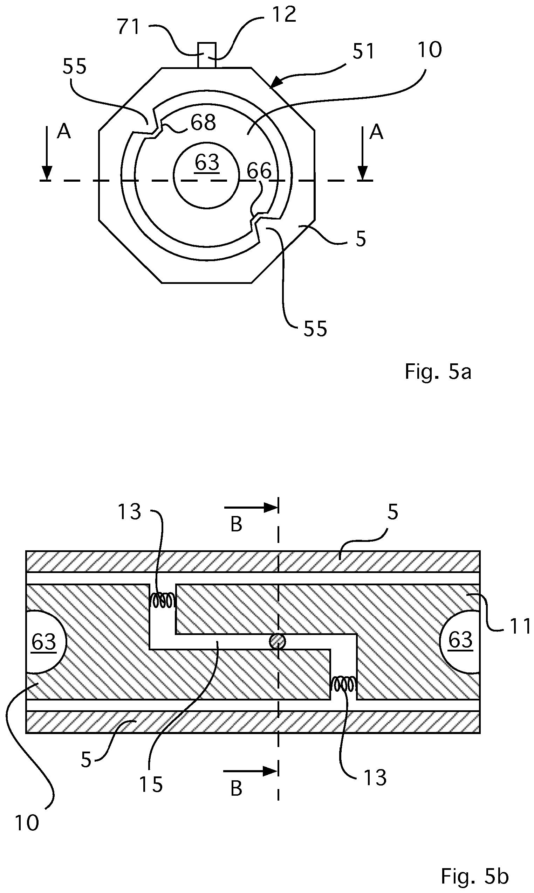

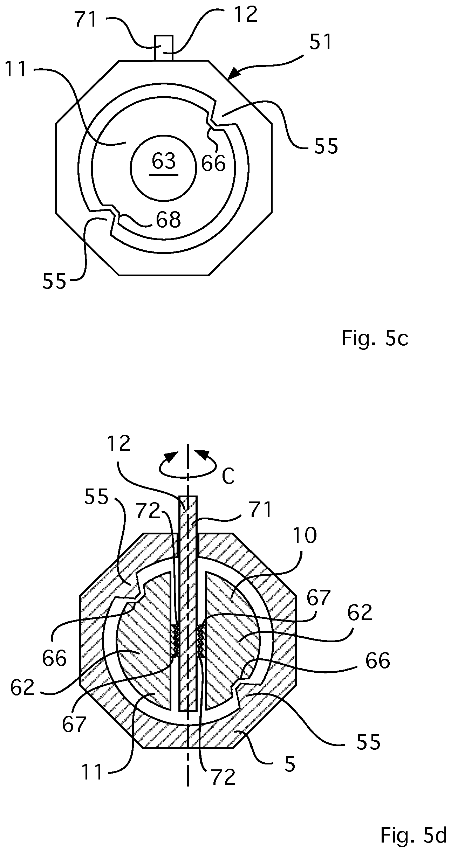

Die Expansionselemente 10, 11 liegen sich in der Hülse 5 gegenüber, wobei die zweiten Abschnitte der beiden Expansionselemente 10, 11 einander zugewandt sind (Fig. 2). Dabei liegen die beiden Flachseiten 64 der Expansionselemente 10, 11 einander gegenüber. Zwischen den Flachseiten 64 der beiden Expansionselemente 10, 11 ist ein Zwischenraum 15 ausgebildet, in dem das Stellelement 12 angeordnet ist. Das Stellelement 12 kann um eine Drehachse (siehe Pfeil C in Fig. 2) gedreht werden, die orthogonal zur Längsachse A der Hülse 5 verläuft. Das Stellelement 12 kann einen Zahnkranz 72 tragen, der in die Zahnreihen 67 der beiden Expansionselemente 10, 11 eingreift (siehe Fig. 5d). Durch Drehen des Stellelementes 12 um seine Drehachse (Pfeil C) werden die Expansionselemente 10, 11 entlang der Längsachse A der Hülse 5 verschoben. Zur transversalen Erweiterung des Oberkiefers des Patienten kann eine Verschiebung des Expansionselementes 10 in Richtung des Pfeiles A und eine Verschiebung des Expansionselementes 11 in Richtung des Pfeiles B durch Drehen des Stellelementes 12 erreicht werden. Dabei vergrößert sich der Abstand zwischen den beiden Expansionselementen 10, 11 in der Hülse. Zur Unterstützung dieser Verschiebung sind Federelemente 13, beispielsweise Druckfedern, zwischen den Expansionselementen 10, 11 angeordnet. In dieser Ausführungsform sind zwei Federelemente 13 vorgesehen. Die Federelemente können sich dabei zwischen der Stirnseite des zweiten Abschnittes 62 eines Expansionselementes 10, 11 und dem Überstand 65 des anderen Expansionselementes 11, 10 erstrecken. Das Stellelement kann einen Stift 71 aufweisen, der durch eine Öffnung in der Hülse geführt ist, um dem Anwender des Distraktors die Verschiebung der Expansionselemente 10, 11 zu ermöglichen.

Die Kopplungselemente 6, 7, 8 weisen jeweils einen Steg 81 auf, der beispielsweise einen kreisförmigen Querschnitt hat und an dessen einer Stirnseite eine Kugelpfanne 82 und an dessen anderer Stirnseite ein Gelenkkopf 83 ausgebildet ist. Der Gelenckopf 83 des Kopplungselementes 6 liegt in der Kugelpfanne 63 des Expansionselementes 10, wodurch ein zweites Kugelgelenk erhalten wird. Der Gelenkkopf 83 des Kopplungselementes 7 liegt in der Kugelpfanne 63 des Expansionselementes 11, wodurch ebenfalls ein zweites Kugelgelenk erhalten wird. Der Gelenkkopf 83 des Kopplungselementes 8 liegt in einer Kugelpfanne 91 eines Trägers 14, wodurch ein viertes Kugelgelenk erhalten wird (siehe Fig. 2).

Die Fixierungselemente 2, 3, 4 sind Schrauben, die jeweils einen konischen Steg 31 aufweisen. Der Steg 31 trägt ein Gewinde und endet in einem Kopf, der als Gelenckopf 32 dient und einen Schlitz 33 oder ein anderes Führungselement für ein Werkzeug aufweist, mit dessen Hilfe die Schrauben in den Oberkiefer des Patienten eingeschraubt werden können. Der Gelenkkopf 32 des ersten Fixierungselementes 2 liegt in der Kugelpfanne 82 des Kopplungselementes 6, wodurch ein erstes Kugelgelenk erhalten wird. Der Gelenkkopf 32 des ersten Fixierungselementes 3 liegt in der Kugelpfanne 82 des Kopplungselementes 7, wodurch ein erstes Kugelgelenk erhalten wird. Der Gelenkkopf 32 des zweiten Fixierungselementes 4 liegt in der Kugelpfanne 82 des Kopplungselementes 8, wodurch ein drittes Kugelgelenk erhalten wird.

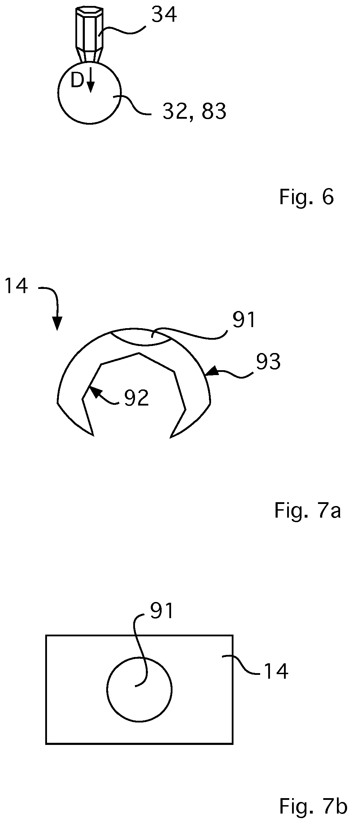

Zur Fixierung der Gelenkköpfe 32, 83 in den Kugelpfannen 63, 82, 91 können Feststellelemente 34, beispielsweise Schrauben wie Stell- oder Kantschrauben vorgesehen sein (siehe Fig. 6). Dazu sind in den Seitenwänden der Kugelpfannen Bohrungen, beispielsweise Versenkbohrungen, ausgebildet, die ein Innengewinde aufweisen, durch die das Feststellelement 34 mit einem korrespondieren Außengewinde geführt werden kann. Das Feststellelement 34 kann dann in die Bohrung eingeschraubt werden, wodurch es mit einer Stirnseite gegen den Gelenkkopf 32, 83 gepresst wird (Pfeil D in Fig. 6). Auf diese Weise kann der Gelenkkopf 32, 83 in der Kugelpfanne 63, 82, 91 starr fixiert werden. Zum Lösen der Fixierung kann das Feststellelement 34 wieder aus der Bohrung geschraubt werden.

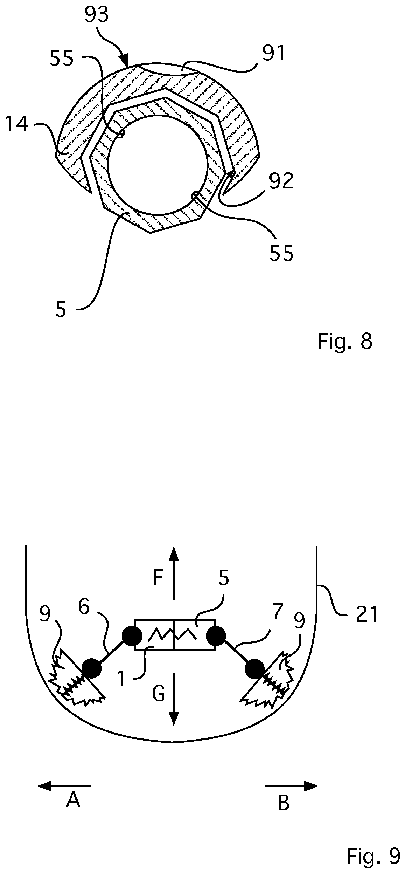

Der Träger 14, der das zweite Kopplungselement 8 und das zweite Fixierungselement 4 an der Hülse hält, kann ein Halbring sein, dessen Innenseite 92 einen Querschnitt aufweist, der komplementär zum äußeren Querschnitt der Hülse 5 ist (siehe Fig. 8). Der Träger 14 kann somit auf die Hülse 5 aufgesteckt werden (siehe Fig. 9). Er ist um die Hülse 5 schwenkbar. Die Kanten 52 der Hülse 5 können zur Festlegung der Position des Trägers 14 auf der Hülse 5 genutzt werden. Die Position des Trägers 14 an der Hülse 5 kann verändert werden, indem der Träger um die Längsachse A der Hülse gedreht wird, bevor er auf die Hülse 5 aufgeschoben wird. An der Außenseite 93 des Trägers 14 ist die Kugelpfanne 91 zur Aufnahme des Gelenkkopfes 83 des zweiten Kopplungselementes 8 ausgebildet.

Die Kugelgelenke des erfindungsgemäßen Distraktors 1 erlauben seine Anpassung in alle Raumrichtungen. In Fig. 9, die einen Querschnitt des Gaumens zeigt, ist zu erkennen, dass der Distraktor 1 in Höhenrichtung (Pfeile F und G) verstellbar ist. Der Träger 14, das zweite Kopplungselement 8 und das zweite Fixierungselement 4 sind in Fig. 9 nicht gezeigt. Zur Anpassung des Distraktors 1 können eines oder mehrere Feststellelemente 34 gelöst, das oder die Gelenke, an denen das oder die Feststellelemente 34 gelöst wurden, neu ausgerichtet und anschließend das oder die Feststellelemente 34 erneut eingeschraubt werden. Das erlaubt neben einer Höhenverstellbarkeit auch einen Symmetrieausgleich. Ein solcher Ausgleich ist insbesondere dann zweckmäßig, wenn aufgrund der Distraktion eines der beiden mit den Expansionselemente 10, 11 verbundenen ersten Fixierungselemente 2, 3 in wesentlich größerem Maße von der Distraktionsebene abweicht als das andere erste Fixierungselemente 3, 2. Die Feststellelemente 34 ermöglichen somit eine Verstellung und/oder Nachjustierung des Distraktors 1. Die Verstellung bzw. Nachjustierung kann sowohl entlang einer Achse, auf der die Pfeile A und B liegen, als auch entlang einer Achse, auf der die Pfeile F und G liegen, erfolgen.

### Bezugszeichenliste

- 1: Distraktor
- 2: erstes Fixierungselement
- 3: erstes Fixierungselement
- 4: zweites Fixierungselement
- 5: Hülse
- 6: erstes Kopplungselement
- 7: erstes Kopplungselement
- 8: zweites Kopplungselement
- 9: Distraktorplatte
- 10: Expansionselement
- 11: Expansionselement
- 12: Stellelement
- 13: Federelement
- 14: Träger
- 15: Zwischenraum

- 21: Oberkiefer
- 22: Ostetomielinie
- 23: lateraler Schneidezahn
- 24: Eckzahn
- 25: laterales Segment
- 26: laterales Segment
- 27: zentrales Segment

- 31: Steg
- 32: Gelenkkopf
- 33: Schlitz
- 34: Feststellelement
- 51: Außenseite
- 52: Kante
- 53: Durchgangsöffnung
- 54: Innenseite
- 55: Steg

- 61: zylinderförmiger Abschnitt
- 62: zweiter Abschnitt
- 63: Kugelpfanne
- 64: Flachseite
- 65: Überstand
- 66: erste Nut
- 67: Zahnreihe
- 68: zweite Nut

- 71: Stift
- 72: Zahnkranz

- 81: Steg
- 82: Kugelpfanne
- 83: Gelenkkopf

- 91: Kugelpfanne
- 92: Innenseite
- 93: Außenseite

### Zitierte Literatur

1. Angell EH. Treatment of irregularities of the permanent or adult tooth. Dental Cosmos 1860; 1: 599-600.
2. Habersack K, Karoglan A, Sommer B et al. High-resolution multislice computerized tomography with multiplanar and 3-dimensional reformation imaging in rapid palatal expansion. American journal of orthodontics and dentofacial orthopedics : official publication of the American Association of Orthodontists, its constituent societies, and the American Board of Orthodontics 2007; 131: 776-781.
3. Korbmacher H, Schilling A, Puschel K et al. Age-dependent three-dimensional microcomputed tomography analysis of the human midpalatal suture. Journal of orofacial orthopedics = Fortschritte der Kieferorthopadie : Organ/official journal Deutsche Gesellschaft für Kieferorthopadie 2007; 68: 364-376.
4. Cureton SL, Cuenin M. Surgically assisted rapid palatal expansion: orthodontic preparation for clinical success. American journal of orthodontics and dentofacial orthopedics : official publication of the American Association of Orthodontists, its constituent societies, and the American Board of Orthodontics 1999; 116: 46-59.
5. Kilic E, Kilic B, Kurt G et al. Effects of surgically assisted rapid palatal expansion with and without pterygomaxillary disjunction on dental and skeletal structures: a retrospective review. Oral surgery, oral medicine, oral pathology and oral radiology 2013; 115: 167-174.
6. Pary A, Cal-Neto JP. A simple method to treat asymmetric expansions in threesegment surgically assisted rapid maxillary expansion. Journal of oral and maxillofacial surgery : official journal of the American Association of Oral and Maxillofacial Surgeons 2013; 71: 2130-2136.
7. Schimming R, Feller KU, Herzmann K et al. Surgical and orthodontic rapid palatal expansion in adults using Glassman's technique: retrospective study. The British journal of oral & maxillofacial surgery 2000; 38: 66-69.
8. Woods M, Wiesenfeld D, Probert T. Surgically-assisted maxillary expansion. Australian dental journal 1997; 42: 38-42.
9. Glassman AS, Nahigian SJ, Medway JM et al. Conservative surgical orthodontic adult rapid palatal expansion: sixteen cases. American journal of orthodontics 1984; 86: 207-213.
10. Mommaerts MY. Transpalatal distraction as a method of maxillary expansion. The British journal of oral & maxillofacial surgery 1999; 37: 268-272.
11. Bell WH, Epker BN. Surgical-orthodontic expansion of the maxilla. American journal of orthodontics 1976; 70: 517-528.
12. Lines PA. Adult rapid maxillary expansion with corticotomy. American journal of orthodontics 1975; 67: 44-56.
13. Northway WM, Meade JB, Jr. Surgically assisted rapid maxillary expansion: a comparison of technique, response, and stability. The Angle orthodontist 1997; 67: 309-320.
14. Habersack K, Becker J, Ristow O et al. Dental and skeletal effects of two-piece and three-piece surgically assisted rapid maxillary expansion with complete mobilization: a retrospective cohort study. Journal of oral and maxillofacial surgery : official journal of the American Association of Oral and Maxillofacial Surgeons 2014; 72: 2278-2288.

## Patentansprüche

1. Distraktor, insbesondere zur transversalen Erweiterung eines Kiefers eines Patienten, wobei der Distraktor (1) eine Hülse (5), in der entlang einer Längsachse (A) bewegbare Expansionselemente (10, 11) angeordnet sind, und erste Fixierungselemente (2, 3) zum Befestigen des Distraktors (1) am Kiefer eines Patienten aufweist, wobei die ersten Fixierungselemente (2, 3) über erste Kopplungselemente (6, 7) mit den Expansionselementen (10, 11) verschwenkbar verbunden sind, wobei zwischen den ersten Fixierungselementen (2, 3) und den ersten Kopplungselementen (6, 7) Gelenke ausgebildet sind, **dadurch gekennzeichnet, dass** zwischen den ersten Kopplungselementen (6, 7) und den Expansionselementen (10, 11) Gelenke ausgebildet sind, wobei die Gelenke, über die die ersten Kopplungselemente (6, 7) mit den Expansionselementen (10, 11) verbunden sind, jeweils ein Kugelgelenk, das als zweites Kugelgelenk bezeichnet ist, sind.

2. Distraktor nach Anspruch 1, **dadurch gekennzeichnet, dass** er ein zweites Fixierungselement (4) zum Befestigen des Distraktors (1) am Kiefer eines Patienten aufweist, wobei das zweite Fixierungselement (4) von einem zweiten Kopplungselement (8) gehalten ist und an der Hülse (5) verschwenkbar befestigt ist.

3. Distraktor nach Anspruch 1 oder Anspruch 2, **dadurch gekennzeichnet, dass** die Gelenke, über die die ersten Fixierungselemente (2, 3) mit den ersten Kopplungselementen (6, 7) verbunden sind, jeweils ein Kugelgelenk, das als erstes Kugelgelenk bezeichnet ist, sind.

4. Distraktor nach Anspruch 3, **dadurch gekennzeichnet, dass** das erste Fixierungselement (2, 3) einen Gelenkkopf (32) aufweist, der in einer an dem ersten Kopplungselement (6, 7) ausgebildeten Kugelpfanne (82) gelagert ist.

5. Distraktor nach einem der vorstehenden Ansprüche, **dadurch gekennzeichnet, dass** das erste Kopplungselement (6, 7) einen Gelenkkopf (83) aufweist, der in einer an dem Expansionselement (10, 11) ausgebildeten Kugelpfanne (63) gelagert ist.

6. Distraktor nach einem der vorstehenden Ansprüche, **dadurch gekennzeichnet, dass** die Hülse (5) einen Träger (14) hält, der um die Längsachse (A) der Hülse (5) drehbar ist, wobei das zweite Kopplungselement (8) das zweite Fixierungselement (4) mit dem Träger (14) verbindet.

7. Distraktor nach einem der vorstehenden Ansprüche, **dadurch gekennzeichnet, dass** das zweite Fixierungselement (4) mit dem zweiten Kopplungselement (8) über ein drittes Kugelgelenk verbunden ist.

8. Distraktor nach Anspruch 7, **dadurch gekennzeichnet, dass** das zweite Fixierungselement (4) einen Gelenkkopf (32) aufweist, der in einer an dem zweiten Kopplungselement (8) ausgebildeten Kugelpfanne (82) gelagert ist.

9. Distraktor nach einem der Ansprüche 6 bis 8, **dadurch gekennzeichnet, dass** das zweite Kopplungselement (8) mit dem Träger (14) über ein viertes Kugelgelenk verbunden ist.

10. Distraktor nach Anspruch 9, **dadurch gekennzeichnet, dass** das zweite Kopplungselement (8) einen Gelenkkopf (83) aufweist, der in einer an dem Träger (14) ausgebildeten Kugelpfanne (91) gelagert ist.

11. Distraktor nach einem der Ansprüche 6 bis 10, **dadurch gekennzeichnet, dass** der Träger (14) ein Halbring ist.

12. Distraktor nach einem der vorstehenden Ansprüche, **dadurch gekennzeichnet, dass** in der Hülse (5) zumindest ein Federelement (13) zwischen einander zugewandten Stirnseiten der Expansionselemente (10, 11) angeordnet ist.

13. Distraktor nach einem der vorstehenden Ansprüche, **dadurch gekennzeichnet, dass** zumindest eines der Expansionselemente (10, 11) eine der Innenseite (54) der Hülse (5) zugewandte Mantelfläche aufweist, in der zumindest eine Führungsnut (66) ausgebildet ist, wobei in die Führungsnut (66) ein Steg (55) eingreift, der an der Innenseite (54) der Hülse (5) ausgebildet ist.

14. Distraktor nach einem der vorstehenden Ansprüche, **dadurch gekennzeichnet, dass** er ein Stellelement (12) zur Verschiebung der Expansionselemente (10, 11) entlang der Längsachse (A) der Hülse (5) aufweist.

## Claims

1. A distractor, particularly to transversally extend a jaw of a patient, wherein the distractor (1) has a case (5) in which expansion elements (10, 11) are arranged that are moveable along a longitudinal axis (A), and first fixing elements (2, 3) to attach the distractor (1) to the jaw of a patient, wherein the first fixing elements (2, 3) are pivotally connected to the expansion elements (10, 11) via first coupling elements (6, 7), wherein joints are formed between the first fixing elements (2, 3) and the first coupling elements (6, 7), **characterized in that** joints are formed between the first coupling elements (6, 7) and the expansion elements (10, 11), wherein the joints, via which the first coupling elements (6, 7) are connected to the expansion elements (10, 11), each are a ball joint designated as a second ball joint.

2. The distractor according to claim 1, **characterized in that** it has a second fixing element (4) to attach the distractor (1) to the jaw of a patient, wherein the second fixing element (4) is held by a second coupling element (8) and is pivotally attached to the case (5).

3. The distractor according to claim 1 or claim 2, **characterized in that** the joints, via which the first fixing elements (2, 3) are connected to the first coupling elements (6, 7), each are a ball joint designated as a first ball joint.

4. The distractor according to claim 3, **characterized in that** the first fixing element (2, 3) has a joint head (32) that is mounted in a ball socket (82) formed on the first coupling element (6, 7).

5. The distractor according to any of the preceding claims, **characterized in that** the first coupling element (6, 7) has a joint head (83) that is mounted in a ball socket (63) formed on the expansion element (10, 11).

6. The distractor according to any of the preceding claims, **characterized in that** the case (5) holds a support (14) rotatable about the longitudinal axis (A) of the case (5), wherein the second coupling element (8) connects the second fixing element (4) to the support (14).

7. The distractor according to any of the preceding claims, **characterized in that** the second fixing element (4) is connected to the second coupling element (8) via a third ball joint.

8. The distractor according to claim 7, **characterized in that** the second fixing element (4) has a joint head (32) that is mounted in a ball socket (82) formed on the second coupling element (8).

9. The distractor according to any one of claims 6 to 8, **characterized in that** the second coupling element (8) is connected to the support (14) via a fourth ball joint.

10. The distractor according to claim 9, **characterized in that** the second coupling element (8) has a joint head (83) that is mounted in a ball socket (91) formed on the support (14).

11. The distractor according to any one of claims 6 to 10, **characterized in that** the support (14) is a semicircle.

12. The distractor according to any of the preceding claims, **characterized in that** at least one spring element (13) is arranged in the case (5) between front sides of the expansion elements (10, 11) that face each other.

13. The distractor according to any of the preceding claims, **characterized in that** at least one of the expansion elements (10, 11) has a circumferential surface facing the inner side (54) of the case (5) in which at least one guide slot (66) is formed, wherein a land (55) that is formed on the inner side (54) of the case (5) engages the guide slot (66).

14. The distractor according to any of the preceding claims, **characterized in that** it has an adjusting element (12) to displace the expansion elements (10, 11) along the longitudinal axis (A) of the case (5).

## Revendications

1. Distracteur, notamment pour l'élargissement transversal d'une mâchoire d'un patient, le distracteur (1) comprenant un manchon (5) dans lequel sont disposés des éléments d'expansion (10, 11) mobiles le long d'un axe longitudinal (A), et des premiers éléments de fixation (2, 3) pour la fixation du distracteur (1) sur la mâchoire d'un patient, les premiers éléments de fixation (2, 3) étant reliés de manière pivotante aux éléments d'expansion (10, 11) par l'intermédiaire de premiers éléments de couplage (6, 7), des articulations se formant entre les premiers éléments de fixation (2, 3) et les premiers éléments de couplage (6, 7), **caractérisé en ce que** des articulations sont formées entre les premiers éléments de couplage (6, 7) et les éléments d'expansion (10, 11), les articulations reliées aux éléments d'expansion (10, 11) au moyen des premiers éléments de couplage (6, 7) chacune étant une articulation à rotule, désignée de deuxième articulation à rotule.

2. Distracteur selon la revendication 1, **caractérisé en ce qu'**il comprend un second élément de fixation (4) pour fixer le distracteur (1) à la mâchoire d'un patient, le second élément de fixation (4) étant maintenu par un second élément de couplage (8) et étant fixé de manière pivotante au manchon (5).

3. Distracteur selon la revendication 1 ou la revendication 2, **caractérisé en ce que** les articulations, par le biais desquelles les premiers éléments de fixation (2, 3) sont reliés aux premiers éléments de couplage (6, 7), chacune étant une articulation à rotule, désignée de première articulation à rotule.

4. Distracteur selon la revendication 3, **caractérisé en ce que** le premier élément de fixation (2, 3) présente une tête d'articulation (32), laquelle est supportée dans une coupelle sphérique (82) formée sur le premier élément de couplage (6, 7).

5. Distracteur selon l'une des revendications ci-dessus, **caractérisé en ce que** le premier élément de couplage (6, 7) présente une tête d'articulation (83), laquelle est supportée dans une coupelle sphérique (63) formée sur le élément d'expansion (10, 11).

6. Distracteur selon l'une des revendications ci-dessus, **caractérisé en ce que** le manchon (5) maintient un support (14) qui peut tourner autour de l'axe longitudinal (A) du manchon (5), le second élément de couplage (8) reliant le second élément de fixation (4) au support (14).

7. Distracteur selon l'une des revendications ci-dessus, **caractérisé en ce que** le deuxième élément de fixation (4) est relié au deuxième élément de couplage (8) par une troisième articulation à rotule.

8. Distracteur selon la revendication 7, **caractérisé en ce que** le deuxième élément de fixation (4) présente une tête d'articulation (32), laquelle est supportée dans une coupelle sphérique (82) formée sur le deuxième élément de couplage (8).

9. Distracteur selon l'une des revendications de 6 à 8, **caractérisé en ce que** le deuxième élément de couplage (8) est relié au support (14) par une quatrième articulation à rotule.

10. Distracteur selon la revendication 9, **caractérisé en ce que** le deuxième élément de couplage (8) présente une tête d'articulation (83), laquelle est supportée dans une coupelle sphérique (91) formée sur le support (14).

11. Distracteur selon l'une des revendications de 6 à 10, **caractérisé en ce que** le support (14) est un demi-anneau.

12. Distracteur selon l'une des revendications ci-dessus, **caractérisé en ce qu'**au moins un élément de suspension (13) est disposé dans le manchon (5) entre les faces frontales des éléments d'expansion (10, 11) qui se font face.

13. Distracteur selon l'une des revendications ci-dessus, **caractérisé en ce qu'**au moins un des éléments d'expansion (10, 11) présente une surface externe orientée vers la face intérieure (54) du manchon (5), dans laquelle est formée au moins une rainure de guidage (66), une nervure (55) s'engageant dans la rainure de guidage (66), laquelle nervure est formée sur la face intérieure (54) du manchon (5).

14. Distracteur selon l'une des revendications ci-dessus, **caractérisé en ce qu'**il comprend un actionneur (12) pour le déplacement des éléments d'expansion (10, 11) le long de l'axe longitudinal (A) du manchon (5).
